(19) Europäisches Patentamt
European Patent Office
Office européen des brevets

(11) **EP 2 638 492 B1**

(12) **FASCICULE DE BREVET EUROPEEN**

(45) Date de publication et mention
de la délivrance du brevet:
**27.02.2019 Bulletin 2019/09**

(21) Numéro de dépôt: **11794811.7**

(22) Date de dépôt: **08.11.2011**

(51) Int Cl.:
***G16H 50/20*** *(2018.01)*    ***G06N 5/02*** *(2006.01)*

(86) Numéro de dépôt international:
**PCT/FR2011/052605**

(87) Numéro de publication internationale:
**WO 2012/062997 (18.05.2012 Gazette 2012/20)**

(54) **DISPOSITIF DE DÉTECTION À CAPTEUR, PROCÉDÉ DE DÉTECTION ET PROGRAMME D'ORDINATEUR CORRESPONDANTS**

SENSORBASIERTER DETEKTOR, ENTSPRECHENDES NACHWEISVERFAHREN UND COMPUTERPROGRAMM

SENSOR-BASED DETECTION DEVICE, CORRESPONDING DETECTION METHOD AND COMPUTER PROGRAM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.11.2010  FR 1059294**

(43) Date de publication de la demande:
**18.09.2013  Bulletin 2013/38**

(73) Titulaire: **Commissariat à l'Énergie Atomique et aux Énergies Alternatives
75015 Paris (FR)**

(72) Inventeur: **JALLON, Pierre
38100 Grenoble (FR)**

(74) Mandataire: **Bonnet, Michel
Cabinet Bonnet
93, rue Réaumur - Boîte 10
75002 Paris (FR)**

(56) Documents cités:
**DE-A1-102007 063 008**

• **OLIVER N ET AL: "Layered representations for learning and inferring office activity from multiple sensory channels", COMPUTER VISION AND IMAGE UNDERSTANDING, ACADEMIC PRESS, US, vol. 96, no. 2, 1 novembre 2004 (2004-11-01), pages 163-180, XP004583829, ISSN: 1077-3142, DOI: DOI:10.1016/J.CVIU.2004.02.004**
• **PIERRE JALLON: "A Bayesian approach for epileptic seizures detection with 3D accelerometers sensors", 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, 31 août 2010 (2010-08-31), pages 6325-6328, XP031794974, ISBN: 978-1-4244-4123-5**
• **JALLON P ET AL: "Detection system of motor epileptic seizures through motion analysis with 3D accelerometers", 2009 ANNUAL INTERNATIONAL CONFERENCE OF THE IEEE ENGINEERING IN MEDICINE AND BIOLOGY SOCIETY : EMBC 2009 ; MINNEAPOLIS, MINNESOTA, USA, 3 - 6 SEPTEMBER 2009, IEEE, PISCATAWAY, NJ, USA, 3 septembre 2009 (2009-09-03), pages 2466-2469, XP031639459, ISBN: 978-1-4244-3296-7 cité dans la demande**

**Description**

La présente invention concerne un dispositif de détection d'au moins une situation parmi un ensemble de situations prédéterminées dans lesquelles est susceptible de se trouver un système physique observé par au moins un capteur, à partir de données d'observation du système physique fournies par le capteur. Elle concerne également un procédé et un programme d'ordinateur correspondants.

On entend par « système physique », tout système produisant une sortie physique observable par un capteur, le système étant supposé a priori pouvoir se trouver dans un nombre prédéterminé de situations modélisées par le dispositif de détection.

Le système physique observé peut par exemple être un objet inanimé, tel qu'une structure dont on souhaite surveiller l'état pour détecter d'éventuelles anomalies ou déformations à l'aide d'un ou plusieurs capteurs.

Il peut aussi s'agir d'un système animé, tel qu'une personne ou un animal, par exemple souffrant d'une maladie chronique à situations de crises détectables à l'aide d'un capteur. Selon le ou les capteurs utilisés, les situations détectables sont variables et les applications multiples.

L'invention s'applique plus particulièrement à un dispositif de détection comportant :

- au moins un capteur pour la fourniture d'une séquence de données d'observation du système physique,
- des moyens de stockage d'au moins un modèle statistique associant des valeurs possibles des données d'observation aux situations prédéterminées, et
- un calculateur, relié au capteur et aux moyens de stockage, programmé pour sélectionner au moins l'une des situations, parmi la pluralité de situations prédéterminées, à partir de la séquence de données d'observation et dudit au moins un modèle statistique.

En particulier, une application prometteuse fait l'objet de l'article de P. Jallon et al, intitulé « Detection system of motor epileptic seizures through motion analysis with 3d accelerometers," publié lors de la conférence IEEE EMBC 2009. Dans cet article, un dispositif de détection de crises d'épilepsie utilisant des capteurs de mouvements, notamment des accéléromètres 3D, est basé sur des modèles statistiques de Markov à états cachés, chacun modélisant au mieux, pour une situation donnée, les propriétés statistiques de séquences d'observation fournies par les capteurs telles qu'elles sont attendues pour cette situation. Concrètement, chaque modèle statistique de Markov à états cachés de ce dispositif correspond à une situation possible prédéterminée d'une personne sujette à des crises d'épilepsie parmi, par exemple : une première situation de crise, une seconde situation de crise différent de la première, une situation d'absence de crise. Le principe de la détection consiste alors à sélectionner l'une des situations possibles, par comparaison de probabilités de ces situations, connaissant une séquence d'observation fournie par au moins un accéléromètre, les probabilités étant calculées sur la base de chacun des modèles statistiques de Markov à états cachés du dispositif.

L'exemple précité fait partie d'une première famille d'application des modèles de Markov à états cachés à la détection de situations, dans laquelle chaque situation est modélisée par un modèle de Markov dont les paramètres sont prédéfinis pour refléter au mieux cette situation. Selon cette première famille d'applications, il est possible de déterminer la situation la plus adaptée à une séquence de données d'observation acquise en sélectionnant le modèle de Markov qui correspond le mieux à cette dernière.

Selon une autre famille d'application des modèles de Markov à états cachés à la détection de situation, telle que par exemple mentionnée dans l'article de L. Rabiner, intitulé "A tutorial on Hidden Markov Models and selected applications in speech récognition," Proceedings of the IEEE, vol. 77, no. 2, pp. 257-286, février 1989, un seul modèle statistique est utilisé, dans lequel chaque état caché correspond à l'une des situations de l'ensemble de situations prédéterminées. Les paramètres du modèle statistique sont alors définis en fonction d'une connaissance a priori de probabilités de chaque situation/état indépendamment de toute observation, de probabilités de transitions d'une situation/état à une autre et de lois probabilités de l'observation pour chaque situation/état. Selon cette seconde famille d'applications, il est possible de déterminer une séquence de situations/états, dans laquelle chaque situation est sélectionnée parmi l'ensemble de situations prédéterminées, cette séquence étant la plus probable compte tenu d'une séquence de données d'observation acquise. La détermination de la séquence de situations/états à partir de la séquence de données d'observation peut par exemple se faire à l'aide de l'algorithme de Viterbi.

On remarque donc que les deux familles d'applications précitées sont liées à des résolutions de problèmes de natures différentes. Cependant les exemples précités ont en commun la propriété de relier directement l'observation dans son ensemble à la décision relative aux situations (sélection d'une situation ou détermination d'une séquence de situations/états) à l'aide du ou des modèle(s) statistique(s) prédéfini(s).

Ainsi, dans le cas où l'observation est multivaluée, c'est-à-dire lorsqu'à chaque instant plusieurs paramètres d'observation sont fournis à l'aide d'un ou de plusieurs capteurs, les dispositifs de détection connus sont particulièrement sensibles aux insuffisances au moins partielles ou temporaires de l'une des sources de paramètres, que cette source soit un capteur ou une chaîne de traitement particulière de signaux issus d'un capteur. De telles insuffisances affectent en effet directement la décision.

En outre, les dispositifs de détection connus sont peu souples en ce qu'ils s'adaptent difficilement à l'ajout ou à la suppression d'un capteur ou d'une chaîne de traitement particulière de signaux issus d'un capteur.

Il peut ainsi être souhaité de prévoir un dispositif de détection qui permette de s'affranchir d'au moins une partie des problèmes et contraintes précités.

L'invention a donc pour objet un dispositif de détection du type précité, dans lequel les moyens de stockage comportent une pluralité de modèles statistiques répartis en plusieurs niveaux ordonnés entre :

- un premier niveau, dit niveau d'entrée, dans lequel chaque modèle statistique associe des valeurs possibles d'au moins une partie des données d'observation à des états intermédiaires propres à ce modèle statistique, et
- un dernier niveau, dit niveau de sortie, dans lequel chaque modèle statistique associe des valeurs possibles d'au moins une partie des états intermédiaires d'un niveau inférieur à au moins une partie des situations prédéterminées.

Ainsi, les données d'observations ne sont qu'indirectement associées aux situations possibles, par l'intermédiaire d'états internes du dispositif propres aux modèles statistiques prédéfinis et stockés en mémoire selon au moins deux niveaux ordonnés. Le niveau d'entrée permet notamment, avec les états intermédiaires, de remplir une fonction de tampon entre les observations et la décision relative aux situations, pour pallier les insuffisances précitées.

En outre, la souplesse du dispositif de détection résultant se trouve améliorée du fait que l'ajout d'un capteur ou d'une chaîne de traitement particulière peut n'affecter directement qu'un modèle statistique du niveau d'entrée, sans pour autant perturber la détermination faite le calculateur sur la base du ou des modèle(s) du niveau de sortie.

Le calculateur est programmé pour :

- déterminer une séquence de valeurs de mesure normalisées d'au moins une partie des états intermédiaires à partir d'au moins un modèle statistique défini pour déterminer cette partie des états intermédiaires et de valeurs que ce modèle statistique reçoit, et
- sélectionner ladite au moins une situation, à partir de la séquence de valeurs de mesure normalisées et d'au moins un modèle statistique du niveau de sortie.

Par « valeurs de mesure normalisées », on entend, pour un modèle statistique donné, que la somme à chaque instant de ses valeurs de mesure d'états intermédiaires, chacune étant de préférence positive, est égale à une constante indépendante du temps. En d'autres termes, une valeur de mesure d'un état intermédiaire déterminable par un modèle statistique donné est dite « normalisée » si la somme des valeurs de mesure de tous les états intermédiaires déterminables par ce modèle statistique et au même instant est égale à une constante choisie et indépendante du temps.

En normalisant la détermination de valeurs d'états intermédiaires qui servent d'observations pour au moins un modèle statistique du niveau de sortie, on fournit en outre l'avantage d'éviter au moins partiellement que certains paramètres d'observation fournis par le capteur n'en occultent d'autres, par exemple par le simple fait qu'ils prennent des valeurs dans des plages d'échelles différentes.

De façon optionnelle, le calculateur est programmé pour déterminer une séquence de valeurs de mesure normalisées de tous les états intermédiaires à partir de tous les modèles statistiques définis pour déterminer ces états intermédiaires et de valeurs que ces modèles statistiques reçoivent.

Il est ainsi possible d'ajuster a priori l'importance relative que l'on souhaite donner à chaque modèle statistique défini pour déterminer des états intermédiaires. En particulier, il est possible d'attribuer la même constante pour toutes les sommes de valeurs de mesures associées à tous les modèles statistiques concernés. Cela procure alors l'avantage de mettre au même niveau tous les paramètres d'observation.

De façon optionnelle également, les valeurs de mesure normalisées sont des valeurs de probabilités. Ceci représente le cas particulier où toutes les valeurs de mesure sont positives et normalisées à la valeur 1.

De façon optionnelle également, au moins un modèle statistique de niveau autre que le niveau de sortie est un modèle de Markov à états cachés et le calculateur est programmé pour déterminer de façon itérative une séquence de valeurs de probabilités d'au moins une partie de ses états cachés à partir d'une séquence de données fournies en entrée de ce modèle de Markov selon une relation de récurrence entre valeurs successives de la séquence de valeurs de probabilités, cette relation de récurrence faisant intervenir une unique valeur, dépendante de l'indice de récurrence, de la séquence de données fournies en entrée.

De façon optionnelle également, la relation de récurrence prend la forme suivante :

INITIALISATION :

$$\tilde{p}\left(X_0 = i \middle| Y_0\right) = p\left(X_0 = i\right) p\left(Y_0 \middle| X_0 = i\right)$$

et

$$p\left(X_0 = i \middle| Y_0\right) = \frac{\tilde{p}\left(X_0 = i \middle| Y_0\right)}{\sum_j \tilde{p}\left(X_0 = j \middle| Y_0\right)},$$

RECURRENCE SUR N :

$$\tilde{p}\left(X_{N+1} = i \middle| Y_{0:N+1}\right) = \sum_j p\left(X_N = j \middle| Y_{0:N}\right) p\left(Y_{N+1} \middle| X_{N+1} = i\right) p\left(X_{N+1} = i \middle| X_N = j\right)$$

et

$$p\left(X_{N+1} = i \middle| Y_{0:N+1}\right) = \frac{\tilde{p}\left(X_{N+1} = i \middle| Y_{0:N+1}\right)}{\sum_j \tilde{p}\left(X_{N+1} = j \middle| Y_{0:N+1}\right)},$$

où i représente l'indice de l'état caché considéré, N l'indice de séquence, Y le processus statistique représentant la séquence de données fournies en entrée, X le processus statistique correspondant à la séquence d'états cachés la plus adaptée à la séquence de données d'entrée.

De façon optionnelle également :

- le niveau de sortie comporte un modèle statistique de Markov dont les états cachés correspondent aux situations prédéterminées et dont les observations sont choisies parmi des valeurs d'états intermédiaires estimées et fournies par le calculateur, et
- le calculateur est programmé pour fournir en sortie une séquence de situations, chacune sélectionnée parmi les situations prédéterminées, à partir de la séquence de valeurs d'états intermédiaires et du modèle statistique de Markov du niveau de sortie.

De façon optionnelle également :

- le niveau de sortie comporte un modèle statistique de Markov à états cachés pour chaque situation de l'ensemble de situations prédéterminées, dont les observations sont choisies parmi des valeurs d'états intermédiaires estimées et fournies par le calculateur, et
- le calculateur est programmé pour sélectionner un modèle de Markov du niveau de sortie à partir de la séquence de valeurs d'états intermédiaires et pour fournir en sortie la situation correspondante.

De façon optionnelle également, au moins un modèle statistique de niveau autre que le niveau d'entrée et le niveau de sortie est un modèle de Markov à états cachés et à observations choisis au moins en partie parmi les états intermédiaires.

De façon optionnelle également, au moins un modèle statistique est à observations choisies en partie parmi les valeurs de données d'observation et en partie parmi des valeurs d'états intermédiaires d'un autre modèle statistique estimées et fournies par le calculateur.

L'invention a également pour objet un procédé de détection d'au moins une situation parmi un ensemble de situations prédéterminées dans lesquelles est susceptible de se trouver un système physique observé par au moins un capteur, à partir de données d'observation du système physique fournies par le capteur, comportant les étapes suivantes :

- réception d'une séquence de données d'observation du système physique fournie par le capteur,
- sélection d'au moins l'une des situations, parmi la pluralité de situations prédéterminées, à partir de la séquence de données d'observation et d'au moins un modèle statistique stocké en mémoire et associant des valeurs possibles des données d'observation aux situations prédéterminées,

et selon lequel, la mémoire stockant une pluralité de modèles statistiques répartis en plusieurs niveaux ordonnés, la sélection comporte les étapes suivantes :

- détermination de valeurs d'états intermédiaires à partir de la séquence de données d'observation et d'au moins un modèle statistique d'un premier niveau, dit niveau d'entrée, dans lequel chaque modèle statistique associe des valeurs possibles d'au moins une partie des données d'observation à des états intermédiaires propres à ce modèle statistique, et
- sélection de ladite au moins une situation, à partir des valeurs d'états intermédiaires et d'au moins un modèle statistique d'un dernier niveau, dit niveau de sortie, dans lequel chaque modèle statistique associe des valeurs possibles d'au moins une partie des états intermédiaires d'un niveau inférieur à au moins une partie des situations prédéterminées.

Ce procédé comporte en outre les étapes suivantes :

- déterminer une séquence de valeurs de mesure normalisées d'au moins une partie des états intermédiaires à partir d'au moins un modèle statistique défini pour déterminer cette partie des états intermédiaires et de valeurs que ce modèle statistique reçoit, et
- sélectionner ladite au moins une situation, à partir de la séquence de valeurs de mesure normalisées et d'au moins un modèle statistique du niveau de sortie.

L'invention a également pour objet un programme d'ordinateur téléchargeable depuis un réseau de communication et/ou enregistré sur un support lisible par ordinateur et/ou exécutable par un processeur, comprenant des instructions pour l'exécution des étapes d'un procédé de détermination de l'état d'une personne selon l'invention, lorsque ledit programme est exécuté sur un ordinateur.

L'invention sera mieux comprise à l'aide de la description qui va suivre, donnée uniquement à titre d'exemple et faite en se référant aux dessins annexés dans lesquels :

- la figure 1 représente schématiquement la structure générale d'un dispositif de détection selon un premier mode de réalisation de l'invention,
- la figure 2 représente schématiquement la structure générale d'un dispositif de détection selon un second mode de réalisation de l'invention,
- la figure 3 illustre une utilisation particulière du dispositif de détection de la figure 1 ou 2,
- la figure 4 illustre les étapes successives d'un procédé de détection selon un mode de réalisation de l'invention,
- les figures 5, 6, 7 et 8 représentent schématiquement différents agencements possibles de modèles statistiques stockés dans le dispositif de détection de la figure 1 ou 2, pour une première application,
- la figure 9 représente schématiquement un agencement possible de modèles statistiques stockés dans le dispositif de détection de la figure 1 ou 2, pour une seconde application, et
- la figure 10 illustre, à l'aide de diagrammes, le fonctionnement du dispositif de la figure 1 ou 2 pour la seconde application de la figure 9.

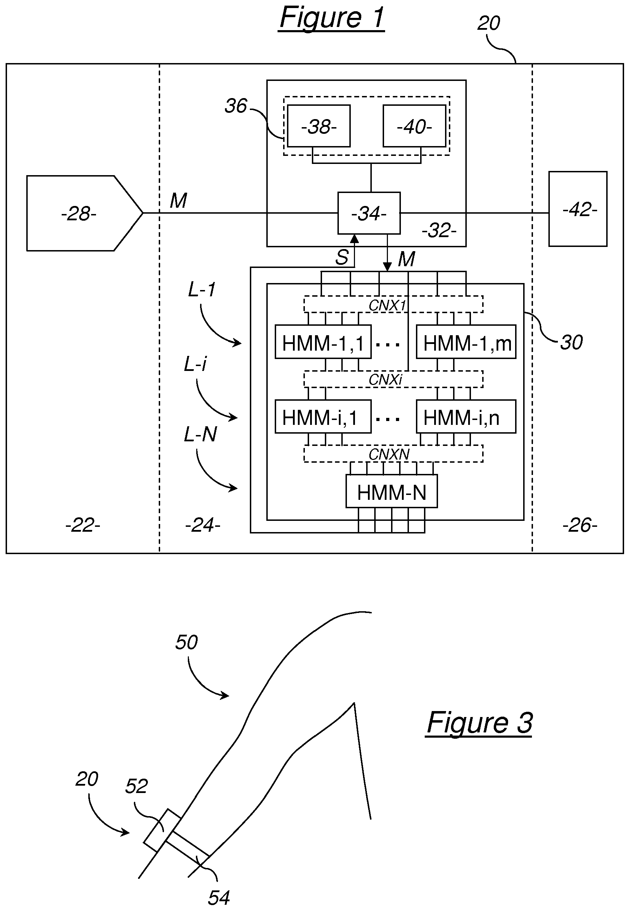

Le dispositif 20 représenté sur la figure 1 est un dispositif de détection d'au moins une situation parmi un ensemble de situations prédéterminées S-1, ..., S-p dans lesquelles est susceptible de se trouver un système physique observé par au moins un capteur. Il comporte à cet effet un module d'observation 22, un module de traitement 24 et un module d'interface 26.

Le module d'observation 22 comporte un ou plusieurs capteurs représentés par l'unique référence 28 pour l'observation du système physique.

De façon non limitative, quelques exemples de capteurs et de situations observables à l'aide de ces capteurs sont donnés :

- le capteur 28 peut par exemple comporter un capteur de mouvements à un, deux ou trois axes de mesure, notamment un accéléromètre 3D porté par une personne, pour la détermination d'une situation de crise ou d'absence de crise d'épilepsie de la personne,
- le capteur 28 peut comporter un capteur de mouvements à un, deux ou trois axes de mesure, notamment un accéléromètre 3D porté par une personne, pour la détermination d'une séquence d'attitudes de cette personne parmi plusieurs attitudes possibles susceptibles de se succéder (par exemple couché, debout/assis, changement de position, marche),

- plus généralement, il peut comporter un capteur de mouvements pour la détermination de l'activité d'un système mobile dans un ensemble d'activités prédéterminées,
- il peut comporter un cardiomètre pour la détermination d'une activité ou d'une succession d'activités d'une personne parmi plusieurs activités possibles (par exemple repos, début d'effort, plateau d'effort court, plateau d'effort long, fin d'effort, repos court),
- il peut comporter un capteur de glycémie d'une personne ou d'un animal souffrant de diabète pour la détermination d'une situation de crise ou d'absence de crise,
- il peut comporter un capteur de pression pour déterminer à chaque instant la situation de fonctionnement (normal, limite, anormal) d'une installation sous pression,
- etc.

Le capteur 28 peut aussi comporter plusieurs capteurs fournissant chacun des observations qui, combinées, permettent d'envisager de détecter des situations plus complexes.

Il effectue des mesures sur le système physique pour fournir au moins un signal d'observation, transmis sous la forme d'une séquence M de données d'observation au module de traitement 24. Les données d'observation peuvent être directement issues d'un échantillonnage du signal d'observation ou obtenues après un ou plusieurs traitements, notamment un ou plusieurs filtrages, de ce signal. On comprend ainsi que les données d'observation peuvent être mono ou multivaluées, y compris lorsque l'on ne dispose que d'un seul capteur 28.

Le module de traitement 24 est un circuit électronique, notamment celui d'un ordinateur. Il comporte des moyens de stockage 30, par exemple une mémoire de type RAM, ROM ou autre, dans lesquels sont stockés les paramètres de modèles statistiques, par exemple des modèles de Markov à état cachés.

D'une façon générale, l'ensemble structuré de ces modèles statistiques, stocké en mémoire 30, associe des valeurs possibles des données d'observation aux situations prédéterminées S-1, ..., S-p.

Le module de traitement 24 comporte en outre un calculateur 32, par exemple une unité centrale d'ordinateur munie d'un microprocesseur 34 et d'un espace de stockage d'au moins un programme d'ordinateur 36. Ce calculateur 32, et plus particulièrement le microprocesseur 34, est relié au capteur 28 et à la mémoire 30.

Le programme d'ordinateur 36 remplit deux principales fonctions illustrées par des modules 38 et 40 sur la figure 1.

La première fonction, remplie par le module de détection 38, par exemple sous la forme d'une boucle d'instructions, est une fonction de détection d'au moins une situation dans laquelle se trouve le système physique, sur réception d'une séquence M de données d'observation fournie par le capteur 18. Plus précisément, le module de détection 28 est programmé pour permettre au microprocesseur 34 de sélectionner au moins l'une des situations S-1, ..., S-N par traitement de la séquence M à l'aide de l'ensemble structuré de modèles statistiques stocké en mémoire 30.

La deuxième fonction, remplie par le module 40, par exemple sous la forme d'une boucle d'instructions, est une fonction de post-traitement. Plus précisément, le module de post-traitement 40 peut être programmé pour permettre au microprocesseur 34 de traiter le résultat fourni par l'exécution du module de détection 38, par exemple via des techniques bayésiennes sur des périodes temporelles bien définies. En particulier, ces techniques bayésiennes peuvent être basées sur des modèles de Markov à états cachés conformes à la première famille d'applications précitées. La deuxième fonction est optionnelle et peut par exemple être exécutée dès la fin d'exécution de la première.

Le module d'interface 26 comporte une interface 42 permettant d'afficher le résultat d'une détection réalisée par le microprocesseur 34. Un des intérêts possibles du dispositif de détection 20 étant de détecter au moins une situation critique parmi un ensemble de situations possibles, Cette interface 42 peut notamment comporter un déclencheur d'alerte. L'interface 42 ou son déclencheur d'alerte peut par exemple comporter un écran, pour l'affichage de résultats ou d'un message d'avertissement, un haut-parleur, pour l'émission d'un signal sonore, ou un émetteur, pour la transmission d'un signal vers une alarme distante.

L'ensemble de modèles statistiques stocké en mémoire 30 est structuré en au moins deux niveaux ordonnés de modèles statistiques, entre un premier niveau L-1, dit niveau d'entrée, et un dernier niveau L-N, dit niveau de sortie. Dans le niveau d'entrée L-1, chaque modèle statistique associe des valeurs possibles d'au moins une partie des données d'observation à des états intermédiaires propres à ce modèle statistique. En particulier, lorsque la séquence M de données d'observation est multivaluée, elle peut être considérée comme un ensemble de plusieurs séquences de valeurs d'observation, chaque modèle statistique du niveau d'entrée L-1 recevant une ou plusieurs de ces séquences de valeurs d'observation. C'est ce qui est symbolisé sur la figure 1 par l'interface de connexion CNX1 représentée en pointillés entre la séquence M de données d'observation et le niveau d'entrée L-1. Dans le niveau de sortie L-N, chaque modèle statistique associe des valeurs possibles d'au moins une partie des états intermédiaires d'un niveau inférieur à au moins une partie des situations prédéterminées S-1, ..., S-p. C'est ce qui est symbolisé sur la figure 1 par l'interface de connexion CNXN représentée en pointillés en entrée du niveau de sortie L-N.

Selon des variantes de réalisation simples, l'ensemble structuré de modèles statistiques stocké en mémoire 30 peut ne comporter que deux niveaux, le niveau d'entrée et le niveau de sortie. Chaque modèle statistique du niveau de sortie L-N puise alors ses observations parmi des valeurs prises par des états intermédiaires de modèle(s) statistique(s) du niveau d'entrée. Ces états intermédiaires sont des états internes de l'ensemble structuré stocké en mémoire 30 qui permettent à la modélisation statistique du dispositif de détection 20 de remplir une fonction de tampon entre la séquence M de données d'observation et la décision relative aux situations S-1, ..., S-N.

Selon des variantes de réalisation plus complexes, telles qu'illustrées par la figure 1, l'ensemble structuré de modèles statistiques stocké en mémoire 30 peut comporter au moins trois niveaux, le niveau d'entrée L-1, le niveau de sortie L-N et au moins un autre niveau L-i. Chaque modèle statistique d'un niveau quelconque L-i associe des valeurs possibles d'au moins une partie des données d'observation et/ou d'au moins une partie des états intermédiaires d'un niveau inférieur à des états intermédiaires propres à ce modèle statistique. C'est ce qui est symbolisé sur la figure 1 par l'interface de connexion CNXi représentée en pointillés en entrée du niveau L-i.

Dans le premier mode de réalisation illustré sur la figure 1 à titre d'exemple non limitatif, chaque modèle statistique est un modèle de Markov à états cachés, chaque niveau comporte un nombre de modèles statistiques qui peut être différent des autres niveaux et le niveau de sortie L-N comporte un unique modèle statistique. Ainsi, le niveau d'entrée L-1 comporte m modèles de Markov notés HMM-1,1 à HMM-1,m, le niveau L-i représenté comporte n modèles de Markov notés HMM-i,1 à HMM-i,n et le niveau de sortie L-N comporte un modèle de Markov noté HMM-N.

L'un quelconque de ces modèles statistiques de Markov à état cachés stockés, noté de façon générique HMM est défini par les paramètres suivants :

- C, le nombre d'états cachés de ce modèle HMM,
- $\pi_1$, ..., $\pi_C$, les C probabilités initiales, indépendantes de toute observation, de chaque état caché de ce modèle HMM,
- $(a_{i,j})_{1 \leq i,j \leq C}$, la matrice des probabilités de transition de chaque état caché i vers chaque autre état caché j de ce modèle HMM, et
- pour chaque état caché, les paramètres d'une loi de probabilité de l'observation fournie à chaque instant en entrée de ce modèle.

A titre d'exemple non limitatif et pour simplifier les notations, la loi de probabilité de chaque état caché i du modèle HMM peut être choisie dans la famille des lois normales. Dans ce cas, elle est définie par son espérance $\mu_i$ et sa variance $\Sigma_i$. Lorsque les données fournies en entrée du modèle HMM sont multivaluées, $\mu_i$ est un vecteur comportant autant de composantes et $\Sigma_i$ une matrice comportant autant de lignes et de colonnes que de valeurs fournies à chaque instant en entrée du modèle HMM.

Ce premier mode de réalisation est conforme à la seconde famille précitée d'application des modèles de Markov à états cachés à la détection de situations, dans laquelle chaque état caché du modèle HMM-N du niveau de sortie L-N correspond à l'une des situations de l'ensemble de situations prédéterminées S-1, ..., S-p. Selon cette seconde famille d'applications, le microprocesseur 34 retourne, en sortie du modèle HMM-N et de façon avantageuse par exécution de l'algorithme de Viterbi adapté à la situation, une séquence S de situations considérée comme la plus probable compte tenu de la séquence M de données d'observation.

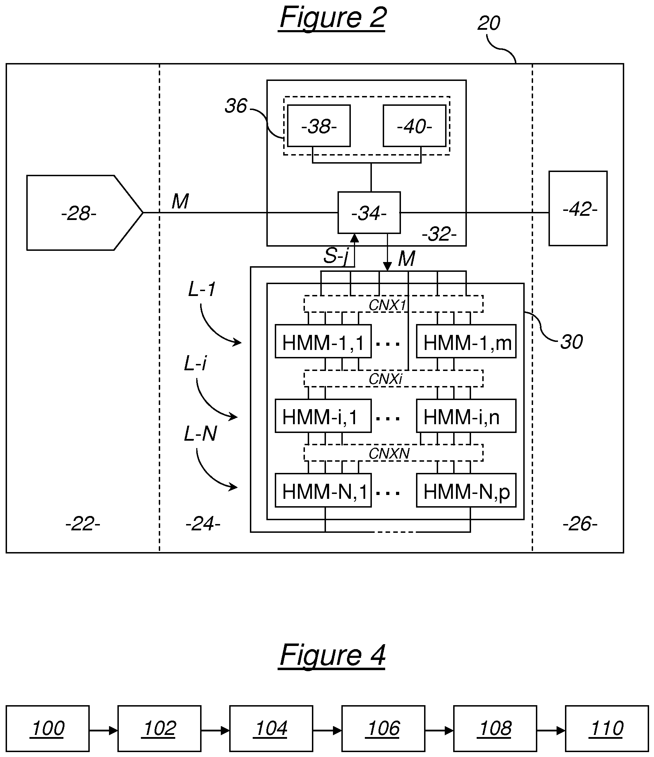

La figure 2 illustre un second mode de réalisation dont les éléments communs avec le premier mode de réalisation de la figure 1 portent les mêmes références et ne seront pas détaillés.

Ce second mode de réalisation diffère du précédent en ce que le niveau de sortie L-N comporte plusieurs modèles de Markov, en particulier un modèle de markov par situation prédéterminée possible, notés HMM-N,1 à HMM-N,p.

Ce second mode de réalisation est conforme à la première famille précitée d'application des modèles de Markov à états cachés à la détection de situations, dans laquelle chaque modèle HMM-N,1, ... HMM-N,p du niveau de sortie L-N correspond à l'une des situations de l'ensemble de situations prédéterminées S-1, ..., S-p, ses paramètres étant prédéfinis pour refléter au mieux cette situation. Selon cette première famille d'applications, le microprocesseur 34 sélectionne, en sortie du niveau L-N, une situation S-j considérée comme la plus représentative de la séquence M de données d'observation. On notera que conformément à cette famille d'applications, les états cachés de chaque modèle statistique du niveau de sortie L-N n'ont pas de signification particulière.

De façon optionnelle, dans les deux modes de réalisation précités, les modèles statistiques de Markov des niveaux L-1 à L-(N-1), c'est-à-dire tous les niveaux autres que le niveau de sortie L-N, présentent des états intermédiaires dont il est possible d'extraire une séquence de valeurs normalisées.

En pratique, le programme 38 exécuté par le microprocesseur 34 est conçu pour :

- déterminer une séquence de valeurs de mesure normalisées d'au moins une partie des états intermédiaires que présentent les modèles statistiques de Markov des niveaux autres que le niveau de sortie L-N, et
- sélectionner la séquence S ou la situation S-j, à partir de la séquence de valeurs de mesure normalisées.

Comme indiqué précédemment, par « valeurs normalisées », on entend, pour un modèle statistique donné, que la somme à chaque instant de ses valeurs de mesure d'états intermédiaires est égale à une constante indépendante du temps.

Dans ce cas, on fournit en outre l'avantage d'éviter au moins partiellement que certains paramètres d'observation fournis par le capteur 28 n'en occultent d'autres, par exemple par le simple fait qu'ils prennent des valeurs dans des plages d'échelles différentes.

De façon optionnelle également, le programme 38 exécuté par le microprocesseur 34 est conçu pour déterminer une séquence de valeurs de mesure normalisées de tous les états intermédiaires à partir de tous les modèles statistiques définis pour déterminer ces états intermédiaires et de valeurs que ces modèles statistiques reçoivent. De préférence, la même constante est attribuée à tous les modèles statistiques concernés pour mettre au même niveau tous les paramètres d'observation.

Les valeurs normalisées sont par exemple des valeurs de probabilités de chacun des états intermédiaires de chaque modèle des niveaux autres que le niveau de sortie L-N en fonction des valeurs fournies en entrée de ce modèle. Un autre avantage de cette normalisation, notamment sous forme de probabilités, est de permettre de définir a priori des valeurs par défaut de chacun des états intermédiaires, de manière à pallier des insuffisances au moins partielles ou temporaires du capteur 28.

On notera enfin que cette option de normalisation n'est pas dépendante de la nature des modèles statistiques utilisés. Dans le cas de modèles de Markov dont les sorties exploitables par le niveau de sortie sont des états cachés des modèles des niveaux inférieurs, l'option de normalisation s'applique à ces états cachés. Mais d'une façon plus générale, cette option de normalisation s'applique aux sorties des modèles statistiques des niveaux inférieurs exploitées par le niveau de sortie, ces sorties étant qualifiées d'états intermédiaires.

Un calcul de probabilités des états intermédiaires va maintenant être détaillé dans le cas particulier mais non limitatif de modèles de Markov à états cachés.

On note HMM la notation générique de l'un des modèles statistiques de l'un des niveaux autres que le niveau de sortie, $Y_n$ le processus statistique représentant une séquence de données fournies en entrée du modèle HMM et $X_n$ le processus statistique correspondant à la séquence d'états cachés la plus adaptée à la séquence de données d'entrée. On suppose en outre que la variable $Y_n$ est indépendante de $Y_{n-1}$ conditionnellement à $X_n$. Cette hypothèse, classique en matière de traitement de signaux à l'aide de modèles de Markov à états cachés, permet d'écrire la relation connue suivante :

$$p\left(X_{0:N},Y_{0:N}\right) = p\left(X_0\right)p\left(Y_0\middle|X_0\right)\prod_{l=1}^{N} p\left(Y_l\middle|X_l\right)p\left(X_l\middle|X_{l-1}\right), \qquad (1)$$

où 0 :N est une séquence échantillonnée entre les instants 0 et N.

Comme indiqué précédemment, on souhaite déterminer une séquence de valeurs de mesure normalisées des états cachés du modèle HMM à partir de la séquence de données d'entrée de ce modèle, notamment par un calcul de séquence de probabilités.

Pour chaque état caché Ei du modèle HMM, on note $p(X_N = i|Y_{0:N})$ sa probabilité à l'instant N au vu de la séquence $Y_{0:N}$.

Cette probabilité se calcule comme suit :

$$p\left(X_N = i\middle|Y_{0:N}\right) = p\left(X_N = i, Y_{0:N}\right)/p\left(Y_{0:N}\right) = \frac{1}{p\left(Y_{0:N}\right)}\sum_{X_0,\dots,X_{N-1}} p\left(X_{0:N-1}, X_N = i, Y_{0:N}\right). \qquad (2)$$

A l'aide de l'équation (1), l'équation (2) devient :

$$p\left(X_N = i\middle|Y_{0:N}\right) = \frac{1}{p\left(Y_{0:N}\right)}\sum_{X_0,\dots,X_{N-1}} p\left(X_0\right)p\left(Y_0\middle|X_0\right)\prod_{l=1}^{N-1} p_l \text{ , où} \qquad (3)$$

$$p_l = p\left(Y_l\middle|X_l\right)p\left(X_l\middle|X_{l-1}\right)p\left(Y_N\middle|X_N = i\right)p\left(X_N = i\middle|X_{N-1}\right).$$

Introduisons à présent la variable $\alpha_0(i) = p(X_0 = i)p(Y_0|X_0 = i)$ et la suite $\alpha_n(i)$ définie par :

$$\alpha_n(i) = \sum_j \alpha_{n-1}(j) p(Y_n | X_n = i) p(X_n = i | X_{n-1} = j). \tag{4}$$

On peut alors montrer que la probabilité $p(X_N = i | Y_{0:N})$ s'exprime comme suit en fonction de la suite $\alpha_n(i)$ :

$$p(X_N = i | Y_{0:N}) = \frac{1}{p(Y_{0:N})} \alpha_N(i) = \frac{\alpha_N(i)}{\sum_i \alpha_N(i)} \tag{5}$$

car la somme des probalités vaut 1.

Le calcul de la probabilité $p(X_N = i | Y_{0:N})$ peut donc se faire de manière itérative grâce aux équations (4) et (5). Ces équations définissent en effet une relation de récurrence entre valeurs successives de la séquence de valeurs de probabilités $p(X_N = i | Y_{0:N})$. En outre, on observe que cette relation de récurrence fait intervenir une unique valeur, dépendante de l'indice de récurrence, de la séquence de données fournies en entrée : il s'agit plus précisément de la dernière donnée fournie en entrée à l'itération N, $Y_N$, qui doit être connue pour déterminer les valeurs de $p(Y_N | X_N = i)$ dans l'expression de $\alpha_N(i)$.

Mais en pratique, des problèmes de convergence numérique peuvent se poser et on préfère utiliser les équations équivalentes suivantes :

INITIALISATION :

$$\tilde{p}(X_0 = i | Y_0) = p(X_0 = i) p(Y_0 | X_0 = i),$$

$$p(X_0 = i | Y_0) = \frac{\tilde{p}(X_0 = i | Y_0)}{\sum_j \tilde{p}(X_0 = j | Y_0)}$$

(valeur normalisée).

RECURRENCE SUR N :

$$\tilde{p}(X_{N+1} = i | Y_{0:N+1}) = \sum_j p(X_N = j | Y_{0:N}) p(Y_{N+1} | X_{N+1} = i) p(X_{N+1} = i | X_N = j),$$

$$p(X_{N+1} = i | Y_{0:N+1}) = \frac{\tilde{p}(X_{N+1} = i | Y_{0:N+1})}{\sum_j \tilde{p}(X_{N+1} = j | Y_{0:N+1})}$$

(valeur normalisée).

Ces équations de récurrence sont équivalentes aux équations (4) et (5) parce que $p(X_N = i | Y_{0:N})$ est proportionnel à $\alpha_n(i)$ et que ce coefficient de proportionnalité est réglé par la normalisation imposant à la somme des probabilités de valoir 1.

On notera que les modules d'observation 22, de traitement 24 et d'interface 26 sont structurellement séparables. Ainsi le dispositif de détection 20 peut être conçu d'un seul tenant ou en plusieurs éléments matériels distincts reliés entre eux par des moyens de transmission de données avec ou sans fil. En particulier, les modules de traitement 24 et éventuellement d'interface 26 peuvent être mis en oeuvre par ordinateur. Seul le module d'observation 22 est nécessairement au voisinage voire au contact du système physique observé puisqu'il comporte le ou les capteurs.

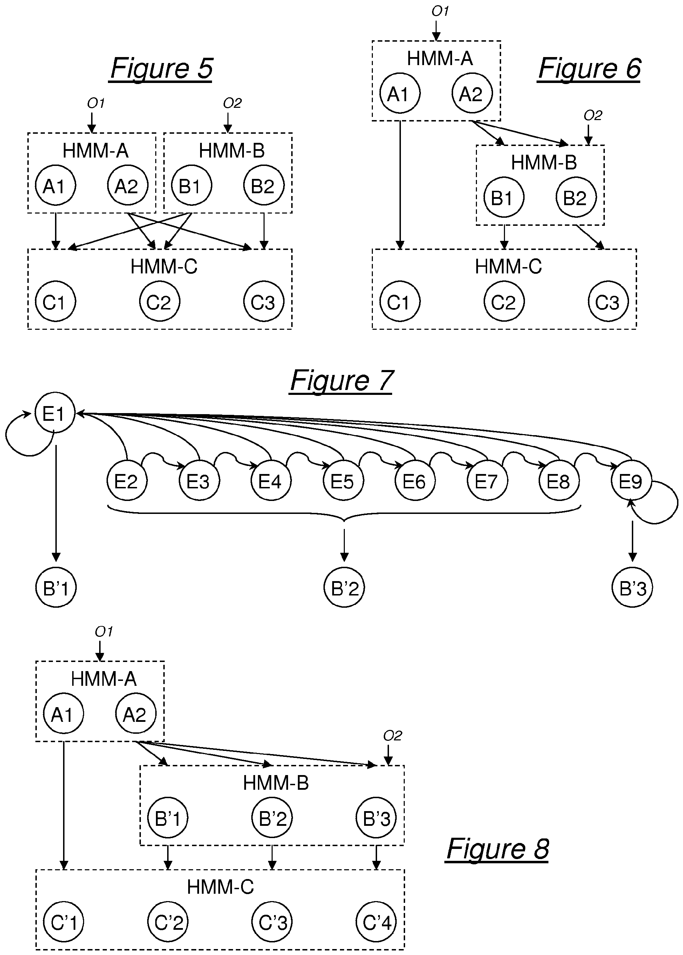

Sur la figure 3, un mode de réalisation particulièrement compact est illustré, pour une application de surveillance d'une personne 50. Selon ce mode de réalisation, le dispositif de détection 20 de la figure 1 ou 2 est tout entier intégré dans un boîtier 52 porté par la personne. Le capteur est par exemple un accéléromètre 3D et les situations observées sont par exemple au nombre de trois comme dans l'exemple particulier qui sera détaillé en référence aux figures 5 et 6 : un état « couché », un état « debout/assis » et un état « en mouvement » de la personne 50, chacun de ces états étant représenté par un état caché correspondant d'un unique modèle de Markov du niveau de sortie L-N. Pour cette application, le boîtier 52 est par exemple fermement maintenu à un bras de la personne 50 au moyen d'un bracelet 54, de sorte que le dispositif de détection 20 est porté tel une montre. De façon encore plus pertinente pour cette application, le boîtier 52 pourrait être maintenu à la taille de la personne 50 à l'aide d'une ceinture.

Le fonctionnement du dispositif de détection 20 va maintenant être détaillé en référence à la figure 4. Plus précisément, l'exécution du module de détection 38 et du module de post-traitement 40 par le microprocesseur 34 produit la séquence d'étapes illustrées sur cette figure.

Au cours d'une première étape 100, le capteur 28 (ou les capteurs en cas de pluralité de capteurs) transmet une séquence M de données d'observation au calculateur 32. Ces données sont par exemple multivaluées de sorte que la séquence de données est en fait une union de séquences de valeurs d'observation, chaque séquence de valeurs étant issue d'un capteur ou d'un traitement spécifique d'un signal fourni par un capteur.

Au cours d'une étape 102, pendant la réception 100 des données successives transmises par le capteur 28, le microprocesseur 34 du calculateur 32 lance l'exécution du module logiciel de détection 38.

Au cours de l'étape 104 suivante, les valeurs de probabilités d'états cachés des modèles statistiques du niveau d'entrée L-1 sont successivement déterminées à partir de la séquence de données d'observation et des modèles statistiques du niveau d'entrée.

Au cours de l'étape 106 suivante, les valeurs de probabilités d'états cachés des modèles statistiques du ou des niveau(x) L-i sont successivement déterminées à partir des valeurs de probabilités d'états cachés des modèles statistiques d'un ou plusieurs niveau(x) inférieur(s), éventuellement également d'une partie de la séquence de données d'observation et des modèles statistiques du niveau L-i.

Au cours d'une étape 108, une séquence S de situations, ou une situation S-j selon que le dispositif 20 met en oeuvre le premier ou le second mode de réalisation, est sélectionnée à partir des valeurs de probabilités d'états cachés des modèles statistiques d'un ou plusieurs niveau(x) inférieur(s) et du ou des modèle(s) statistique(s) du niveau de sortie L-N.

Enfin, au cours d'une étape 110, un post-traitement peut être optionnellement réalisé par le microprocesseur 34 par exécution du module 40 sur un ou plusieurs résultats de l'étape 108.

Il apparaît ainsi clairement que le dispositif de détection 20 décrit précédemment est capable de déterminer, à partir d'une séquence M de données d'observation, une séquence de valeurs d'états intermédiaires des modèles statistiques de niveau autre que le niveau de sortie L-N, de préférence même une séquence de valeurs normalisées telles que des probabilités, puis de déterminer, à partir de cette séquence de valeurs d'états intermédiaires, la situation ou la séquence de situations représentative de la séquence d'observation M, à l'aide respectivement des modèles statistiques ou du modèle statistique du niveau de sortie.

Un premier exemple concret, conforme à la seconde famille d'applications, va maintenant être décrit en référence à la figure 5.

Selon cet exemple, le capteur 28 comporte un capteur de mouvements à un, deux ou trois axes de mesure, notamment un accéléromètre 3D porté par une personne, au niveau de son torse. L'accéléromètre 3D fournit un signal vectoriel qui est filtré et échantillonné pour produire :

- d'une part, une séquence O1 de valeurs d'observation combinant des composantes basses fréquences du signal vectoriel et formant une composante de moyenne de ce signal vectoriel représentative d'une orientation du capteur 28 par rapport au sol,
- d'autre part, une séquence O2 de valeurs d'observation combinant des composantes hautes fréquences du signal vectoriel et formant une composante de variance de ce signal vectoriel représentative d'une dynamique du capteur 28 par rapport au sol.

En pratique et dans cet exemple d'application, les composantes basses fréquences correspondent à des fréquences inférieures à quelques Hertz, par exemple inférieures à 2 Hz. Les composantes hautes fréquences correspondent à des fréquences supérieures à quelques Hertz, par exemple supérieures à 2 Hz.

A un premier niveau d'entrée, les paramètres d'un modèle de Markov à états cachés HMM-A sont stockés dans la mémoire 30. Ce modèle de Markov HMM-A est destiné à recevoir, en entrée, la séquence de valeurs O1 et le microprocesseur 34 est conçu pour fournir, en sortie de ce modèle HMM-A, deux séquences de valeurs de probabilités de deux états cachés A1 et A2. Les probabilités initiales, la matrice des probabilités de transition et les lois de probabilités des états A1 et A2 sont définies et stockées en mémoire 30, pour ce modèle de Markov HMM-A, de telle manière que l'état caché A1 représente un modèle de posture « couché » et que l'état caché A2 représente un modèle de posture « buste droit » de la personne portant le dispositif de détection 20. Il appartient à l'homme de l'art, comme cela est connu en soi, de définir ces paramètres pour que ce modèle statistique HMM-A corresponde au mieux au modèle de posture souhaité pour la personne. Il est notamment possible de modéliser les lois de probabilités des états A1 et A2 par des lois normales de moyennes correspondant aux moyennes des valeurs de la séquence O1 quand la personne est couchée pour l'état A1 et buste droit pour l'état A2.

A ce niveau d'entrée également, les paramètres d'un modèle de Markov à états cachés HMM-B sont stockés dans la mémoire 30. Ce modèle de Markov HMM-B est destiné à recevoir, en entrée, la séquence de valeurs O2 et le microprocesseur 34 est conçu pour fournir, en sortie de ce modèle HMM-B, deux séquences de valeurs de probabilités de deux états cachés B1 et B2. Les probabilités initiales, la matrice des probabilités de transition et les lois de probabilités des états B1 et B2 sont définies et stockées en mémoire 30, pour ce modèle de Markov HMM-B, de telle manière que l'état caché B1 représente un modèle dynamique « statique » et que l'état caché B2 représente un modèle dynamique « en mouvement » de la personne portant le dispositif de détection 20. Il appartient à l'homme de l'art, comme cela est connu en soi, de définir ces paramètres pour que ce modèle statistique HMM-B corresponde au mieux au modèle dynamique souhaité pour la personne. Il est notamment possible de modéliser les lois de probabilités des états B1 et B2 par des lois du $\chi_2$ avec des paramètres dépendant de l'état considéré.

A un second niveau de sortie, les paramètres d'un modèle de Markov à états cachés HMM-C sont stockés dans la mémoire 30. Ce modèle de Markov HMM-C est destiné à recevoir, en entrée, les séquences de valeurs de probabilités des états A1, A2, B1 et B2 et le microprocesseur 34 est conçu pour fournir, en sortie de ce modèle HMM-C, une séquence de situations choisies parmi une situation « couché » représentée par un premier état caché C1 de HMM-C, une situation « debout/assis » représentée par un deuxième état caché C2 de HMM-C et une situation « en train de bouger » représentée par un troisième état caché C3 de HMM-C. Les probabilités initiales, la matrice des probabilités de transition et les lois de probabilités des états C1, C2 et C3 sont définies et stockées en mémoire 30, pour que ce modèle de Markov HMM-C représente de façon réaliste les trois situations précitées. Il appartient à l'homme de l'art, comme cela est connu en soi, de définir ces paramètres pour que ce modèle statistique HMM-C corresponde au mieux au modèle de détermination de situations souhaité pour la personne.

On remarque en outre que, dans l'exemple de la figure 5, les modèles statistiques HMM-A et HMM-B sont indépendants et au même niveau, chacun apportant sa contribution au modèle HMM-C de façon équitable.

Par ailleurs, dans cet exemple la probabilité de l'état caché C1 est définie comme dépendante des probabilités des états cachés A1 et B1. Par conséquent, du fait de l'indépendance des modèles statistiques HMM-A et HMM-B, la probabilité de l'état caché C1 est directement estimée à partir du produit des probabilités des états cachés A1 et B1. La probabilité de l'état caché C2 est définie comme dépendante des probabilités des états cachés A2 et B1. Par conséquent, du fait de l'indépendance des modèles statistiques HMM-A et HMM-B, la probabilité de l'état caché C2 est directement estimée à partir du produit des probabilités des états cachés A2 et B1. Enfin, la probabilité de l'état caché C3 est définie comme dépendante des probabilités des états cachés A2 et B2. Par conséquent, du fait de l'indépendance des modèles statistiques HMM-A et HMM-B, la probabilité de l'état caché C3 est directement estimée à partir du produit des probabilités des états cachés A2 et B2.

Une limite de cet exemple simple où les deux modèles statistiques HMM-A et HMM-B sont indépendants et au même niveau est que, si le capteur 28 bouge alors que la personne est couchée, le dispositif de détection 20 ne saura pas prendre de décision (situation C1 ou C3 ?).

Un deuxième exemple concret pour la même application que le premier est illustré sur la figure 6. Le capteur 28 est identique à celui de l'exemple précédent et fournit les mêmes séquences de valeurs d'observation O1 et O2.

A un premier niveau d'entrée, les paramètres du modèle de Markov à états cachés HMM-A précité sont stockés dans la mémoire 30.

A un deuxième niveau intermédiaire, les paramètres du modèle de Markov à états cachés HMM-B précité sont stockés dans la mémoire 30. Cependant, dans ce deuxième exemple, le microprocesseur 34 est conçu pour fournir, en sortie de ce modèle HMM-B, deux séquences de valeurs de probabilités de deux états cachés B1 et B2 dépendantes de la séquence de valeurs de probabilités de l'état caché A2. Le modèle statistique HMM-B est ainsi dépendant du modèle HMM-A dans ce deuxième exemple.

A un troisième niveau de sortie, les paramètres du modèle de Markov à états cachés HMM-C précité sont stockés dans la mémoire 30. Cependant, dans ce deuxième exemple la probabilité de l'état caché C1 est définie comme dépendante de la probabilité de l'état caché A1 uniquement. La probabilité de l'état caché C2 est définie comme dépendante de la probabilité de l'état caché B1 uniquement. Par conséquent, du fait de la dépendance des modèles statistiques HMM-A et HMM-B, la probabilité de l'état caché C2 est directement estimée à partir de la probabilité de l'état caché B1 si la probabilité de A2 est la plus élevée des états cachés de HMM-A, nulle sinon. Enfin, la probabilité de l'état caché C3 est définie comme dépendante de la probabilité de l'état caché B2 uniquement. Par conséquent, du fait de la dépendance des modèles statistiques HMM-A et HMM-B, la probabilité de l'état caché C3 est directement estimée à partir de la probabilité de l'état caché B2 si la probabilité de A2 est la plus élevée des états cachés de HMM-A, nulle sinon.

Dans ce deuxième exemple contrairement au précédent, si le capteur 28 bouge alors que la personne est couchée, le dispositif de détection 20 décidera que la personne est couchée (situation C1).

Un inconvénient des premier et deuxième exemples mentionnés ci-dessus est que le modèle dynamique HMM-B présente des états cachés non contraints dans le temps, à savoir, soit un état « statique », soit un état « en mouvement ». Il pourrait être souhaité d'affiner ces applications en fournissant un modèle statistique non stationnaire, comportant par exemple un état « statique », un état « en mouvement depuis moins de x secondes » et un état « en mouvement depuis au moins x secondes ». Une solution pour arriver à ce résultat tout en conservant une modélisation de Markov à états cachés est de définir un modèle de Markov avec de nombreux états cachés virtuels, tels que « en mouvement depuis 1 coup d'horloge », « en mouvement depuis 2 coups d'horloge », ..., « en mouvement depuis N coups d'horloge », puis de regrouper certains états cachés virtuels en un état caché réel dont les paramètres sont dérivés de ceux de ces états cachés virtuels.

Un troisième exemple concret pour la même application que le premier et le deuxième est illustré sur la figure 8 et son principe est schématiquement représenté sur la figure 7. Le capteur 28 est identique à celui de l'exemple précédent et fournit les mêmes séquences de valeurs d'observation O1 et O2.

Comme illustré sur la figure 7, le modèle HMM-B est affiné de manière à présenter trois états cachés : l'état caché B'1, identique à B1, représente un modèle dynamique « statique », l'état caché B'2 représente un modèle dynamique « en mouvement depuis moins de 8 coups d'horloge » et l'état caché B'3 représente un modèle dynamique « en mouvement depuis au moins 8 coups d'horloge ». Pour cela, ces trois états sont dérivés de 9 états cachés virtuels E1 à E9.

L'état virtuel E1 correspond à l'état caché B'1. Cet état n'est pas contraint temporellement.

Les états virtuels E2 à E9 sont des états contraints temporellement correspondant aux deux états cachés B'2 et B'3. Si l'on note $V_n$ la variable aléatoire de ces états virtuels, $X_n$ la variable aléatoire de sortie du modèle HMM-B et $Y_n$ la variable aléatoire d'entrée du modèle HMM-B, sachant en outre qu'il est possible de revenir à l'état virtuel E1 de n'importe quel état virtuel, il vient :

$$\forall i \in [2,8], \, p\big(V_n = Ei \big| V_{n-1} = Ej\big) = \begin{cases} 1 \; si \; i = j+1 \\ 1 \; si \; i \neq j+1 \end{cases},$$

$$p\big(V_n = E9 \big| V_{n-1} = Ej\big) = \begin{cases} \lambda \; si \; j = 8 \\ 1 - \lambda \; si \; j = 9 \\ 0 \; si \; j \in [2,7] \end{cases},$$

$$\forall i \in [2,9], \, p\big(Y_n \big| V_{n-1} = Ei\big) = p\big(Y_n \big| V_{n-1} = E2\big),$$

et

$$p\big(X_n = B'1\big) = p\big(V_n = E1\big),$$

$$p\big(X_n = B'2\big) = \sum_{i=2}^{8} p\big(V_n = Ei\big),$$

$$p\big(X_n = B'3\big) = p\big(V_n = E9\big).$$

Ces équations sont aisément généralisables à un ensemble comportant à la fois des états cachés non contraints temporellement et des états cachés contraints temporellement qui dépendent de la même densité de probabilité du vecteur observé.

Il résulte du principe représenté sur la figure 7 l'ensemble structuré de modèles illustré sur la figure 8.

Le premier niveau d'entrée de ce troisième exemple est identique au niveau d'entrée du deuxième exemple.

Au deuxième niveau intermédiaire, le modèle HMM-B est adapté comme indiqué ci-dessus pour présenter les trois états cachés B'1, B'2 et B'3. Le microprocesseur 34 est conçu pour fournir, en sortie de ce modèle HMM-B adapté, trois séquences de valeurs de probabilités des trois états cachés B'1, B'2 et B'3 dépendantes de la séquence de valeurs de probabilités de l'état caché A2.

Au troisième niveau de sortie, le modèle HMM-C est adapté pour présenter quatre états cachés C'1, C'2, C'3 et C'4 : C'1 représente une situation « couché », C'2 une situation« debout/assis », C'3 une situation « changement de position » et C'4 une situation « en train de marcher ». La probabilité de l'état caché C'1 est définie comme dépendante de la probabilité de l'état caché A1 uniquement. La probabilité de l'état caché C'2 est définie comme dépendante de la probabilité de l'état caché B'1 uniquement. Par conséquent, du fait de la dépendance des modèles statistiques HMM-A et HMM-B, la probabilité de l'état caché C'2 est directement estimée à partir de la probabilité de l'état caché B'1 si la probabilité de A2 est la plus élevée des états cachés de HMM-A, nulle sinon. La probabilité de l'état caché C'3 est définie comme dépendante de la probabilité de l'état caché B'2 uniquement. Par conséquent, du fait de la dépendance des modèles statistiques HMM-A et HMM-B, la probabilité de l'état caché C'3 est directement estimée à partir de la probabilité de l'état caché B'2 si la probabilité de A2 est la plus élevée des états cachés de HMM-A, nulle sinon. Enfin, la probabilité de l'état caché C'4 est définie comme dépendante de la probabilité de l'état caché B'3 uniquement. Par conséquent, du fait de la dépendance des modèles statistiques HMM-A et HMM-B, la probabilité de l'état caché C'4 est directement estimée à partir de la probabilité de l'état caché B'3 si la probabilité de A2 est la plus élevée des états cachés de HMM-A, nulle sinon.

Ainsi, on note que la prise en compte d'une contrainte temporelle dans le modèle HMM-B permet en sortie de distinguer la situation « changement de position » de la situation « en train de marcher », ce qui n'était pas le cas dans les deux premiers exemples.

Les trois exemples précités permettent par ailleurs d'envisager un post-traitement spécifique pour prendre une décision plus précise sur la situation C2 = C'2 = « debout/assis ». L'objectif de ce post-traitement peut être de mesurer si le signal de l'accéléromètre 3D est de variance faible inférieure à un seuil prédéterminé auquel cas il peut être considéré que la personne, ne bougeant absolument pas, est assise, ou si le signal de l'accéléromètre 3D est de variance plus élevée supérieure au seuil prédéterminé auquel cas il peut être considéré que la personne, conservant son équilibre à chaque instant, est debout. Ce post-traitement peut être réalisé sur la base d'une modélisation de Markov à états cachés selon la première famille d'applications précitée.

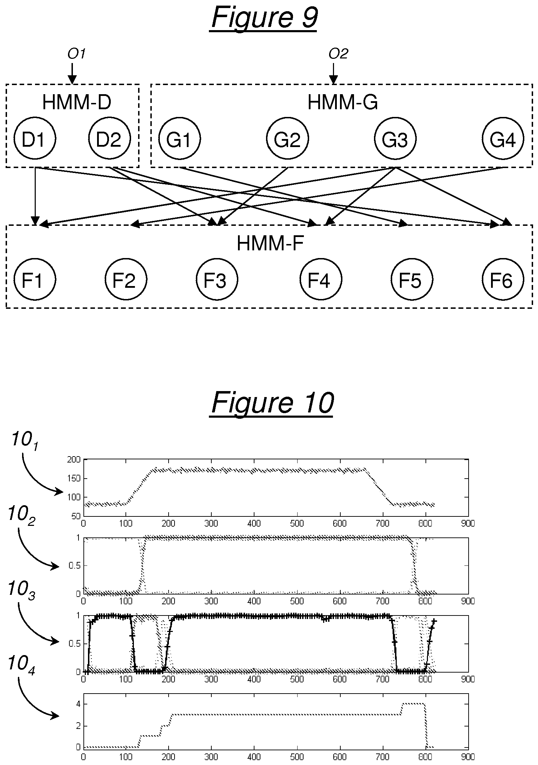

Un quatrième exemple concret, conforme à la seconde famille d'applications, va maintenant être décrit en référence à la figure 9.

Selon cet exemple, le capteur 28 comporte un cardiomètre porté par une personne. Le cardiomètre fournit un signal représentatif des pulsations cardiaques de la personne. Ce signal est traité et échantillonné pour produire :

- d'une part, une séquence O1 de valeurs d'observation obtenue par échantillonnage du signal de pulsations cardiaques, représentative du rythme cardiaque de la personne à chaque instant,
- d'autre part, une séquence O2 de valeurs d'observation obtenue par échantillonnage de la différentielle du signal de pulsations cardiaques, représentative de l'évolution du rythme cardiaque de la personne à chaque instant.

A un premier niveau d'entrée, les paramètres d'un modèle de Markov à états cachés HMM-D sont stockés dans la mémoire 30. Ce modèle de Markov HMM-D est destiné à recevoir, en entrée, la séquence de valeurs O1 et le microprocesseur 34 est conçu pour fournir, en sortie de ce modèle HMM-A, deux séquences de valeurs de probabilités de deux états cachés D1 et D2. Les probabilités initiales, la matrice des probabilités de transition et les lois de probabilités des états D1 et D2 sont définies et stockées en mémoire 30, pour ce modèle de Markov HMM-D, de telle manière que l'état caché D1 représente un modèle de rythme cardiaque « repos » et que l'état caché D2 représente un modèle de rythme cardiaque « élevé » de la personne portant le dispositif de détection 20. Il appartient à l'homme de l'art, comme cela est connu en soi, de définir ces paramètres pour que ce modèle statistique HMM-D corresponde au mieux au modèle de posture souhaité pour la personne. Il est notamment possible de modéliser la loi de probabilité de l'état D1 par une loi normale de moyenne 80 et de variance 20 et de modéliser la loi de probabilité de l'état D2 par une loi normale de moyenne 140 et de variance 30.

A ce niveau d'entrée également, les paramètres d'un modèle de Markov à états cachés HMM-G sont stockés dans la mémoire 30. Ce modèle de Markov HMM-G est destiné à recevoir, en entrée, la séquence de valeurs O2 et le microprocesseur 34 est conçu pour fournir, en sortie de ce modèle HMM-G, quatre séquences de valeurs de probabilités de quatre états cachés G1, G2, G3 et G4. Les probabilités initiales, la matrice des probabilités de transition et les lois de probabilités des états G1, G2, G3 et G4 sont définies et stockées en mémoire 30, pour ce modèle de Markov HMM-G, de telle manière que l'état caché G1 représente un modèle de tendance de rythme cardiaque « en baisse », que l'état caché G2 représente un modèle de tendance de rythme cardiaque « constant inférieur à 10 secondes », que l'état caché G3 représente un modèle de tendance de rythme cardiaque « constant d'au moins 10 secondes » et que l'état caché G4 représente un modèle de tendance de rythme cardiaque « en hausse » de la personne portant le dispositif de détection 20. Il appartient à l'homme de l'art, comme cela est connu en soi, de définir ces paramètres pour que ce modèle statistique HMM-B corresponde au mieux au modèle dynamique souhaité pour la personne. Il est notamment possible de modéliser la loi de probabilité de l'état G1 par une loi normale de moyenne -5 et de variance 3, de modéliser la loi de probabilité de l'état G2 contraint de durer moins de 10 secondes par une loi normale centrée de variance 3, de modéliser la loi de probabilité de l'état G3 contraint de durer au moins 10 secondes par une loi normale centrée de variance 3 et de modéliser la loi de probabilité de l'état G4 par une loi normale de moyenne 5 et de variance 3.

A un second niveau de sortie, les paramètres d'un modèle de Markov à états cachés HMM-F sont stockés dans la mémoire 30. Ce modèle de Markov HMM-F est destiné à recevoir, en entrée, les séquences de valeurs de probabilités des états D1, D2, G1, G2, G3 et G4 et le microprocesseur 34 est conçu pour fournir, en sortie de ce modèle HMM-F, une séquence de situations choisies parmi une situation « repos » représentée par un premier état caché F1 de HMM-F, une situation « début d'effort » représentée par un deuxième état caché F2 de HMM-F, une situation « plateau d'effort court » représentée par un troisième état caché F3 de HMM-F, une situation « plateau d'effort long » représentée par un quatrième état caché F4 de HMM-F, une situation « fin d'effort » représentée par un cinquième état caché F5 de HMM-F et une situation « repos court » représentée par un sixième état caché F6 de HMM-F. Les probabilités initiales, la matrice des probabilités de transition et les lois de probabilités des états F1, F2, F3, F4, F5 et F6 sont définies et stockées en mémoire 30, pour que ce modèle de Markov HMM-F représente de façon réaliste les six situations précitées. Il appartient à l'homme de l'art, comme cela est connu en soi, de définir ces paramètres pour que ce modèle statistique HMM-F corresponde au mieux au modèle de détermination de situations souhaité pour la personne.

On remarque en outre que, dans l'exemple de la figure 9, les modèles statistiques HMM-D et HMM-G sont indépendants et au même niveau, chacun apportant sa contribution au modèle HMM-F de façon équitable.

Par ailleurs, dans cet exemple la probabilité de l'état caché F1 est définie comme dépendante des probabilités des états cachés D1 et G3. Par conséquent, du fait de l'indépendance des modèles statistiques HMM-D et HMM-G, la probabilité de l'état caché F1 est directement estimée à partir du produit des probabilités des états cachés D1 et G3. La probabilité de l'état caché F2 est définie comme dépendante de la probabilité de l'état caché G4 uniquement. La probabilité de l'état caché F3 est définie comme dépendante des probabilités des états cachés D2 et G2. Par conséquent, du fait de l'indépendance des modèles statistiques HMM-D et HMM-G, la probabilité de l'état caché F3 est directement estimée à partir du produit des probabilités des états cachés D2 et G2. La probabilité de l'état caché F4 est définie comme dépendante des probabilités des états cachés D2 et G3. Par conséquent, du fait de l'indépendance des modèles statistiques HMM-D et HMM-G, la probabilité de l'état caché F4 est directement estimée à partir du produit des probabilités des états cachés D2 et G3. La probabilité de l'état caché F5 est définie comme dépendante de la probabilité de l'état caché G1 uniquement. Enfin, la probabilité de l'état caché F6 est définie comme dépendante des probabilités des états cachés D1 et G3. Par conséquent, du fait de l'indépendance des modèles statistiques HMM-D et HMM-G, la probabilité de l'état caché F6 est directement estimée à partir du produit des probabilités des états cachés D1 et G3.

La figure 10 illustre à l'aide de diagrammes un fonctionnement du dispositif de détection 20 dans le contexte de ce quatrième exemple. Le diagramme $10_1$ représente un exemple de signal temporel de pulsations cardiaques fourni par le cardiomètre 28. Le diagramme $10_2$ illustre les séquences de probabilités correspondantes des états cachés D1 et D2. Le diagramme $10_3$ illustre les séquences de probabilités correspondantes des états cachés G1, G2, G3 et G4. Enfin, le diagramme $10_4$ fournit la séquence correspondante de situations choisies parmi les états cachés F1 à F6.

Il apparaît clairement qu'un dispositif de détection de situations tel que celui décrit précédemment permet d'envisager des performances et une souplesse améliorées du fait que les données d'observations ne sont qu'indirectement associées aux situations détectables par le dispositif. L'ajout ou la suppression d'un capteur n'a ainsi qu'un impact relatif sur la modélisation sur laquelle se base le dispositif de détection, celui-ci pouvant n'affecter qu'un modèle statistique du niveau d'entrée. Il est notamment possible de définir un modèle statistique (ou un ensemble de modèles statistiques) de niveau d'entrée spécifique pour chaque capteur.

La possibilité d'attribuer des valeurs par défaut aux états intermédiaires permet en outre d'améliorer la robustesse du dispositif.

On notera par ailleurs que l'invention n'est pas limitée aux modes de réalisation décrits précédemment.

Notamment bien d'autres applications que celles décrites précédemment sont envisageables en fonction des capteurs employés et des modèles définis.

Par exemple, un tel dispositif pourrait également être utilisé pour suivre un taux de glycémie d'une personne et prévenir ou détecter rapidement des situations de crises. Dans ce cas précis, plusieurs éléments sont importants pour déclencher des alarmes d'hypo- ou hyper-glycémie.

Un premier point est bien sûr de mesurer en continu le niveau de glycémie de la personne à l'aide d'un capteur. Si ce niveau dépasse une certaine limite, il est nécessaire d'alerter la personne sur le risque encouru. Un second point est de mesurer la tendance du niveau de glycémie. Un niveau un peu élevé avec une tendance à la hausse faible est moins risqué qu'un niveau moyen/supérieur avec une tendance à la hausse forte. Les modèles présentés dans les exemples précités pourraient donc tout à fait être adaptés à cette application avec deux modèles de niveau d'entrée :

- un premier modèle de Markov utilisant une évaluation du taux de glycémie, à cinq états cachés : glycémie très basse, basse, normale, haute, très haute,
- un second modèle de Markov utilisant une évaluation de l'évolution du taux de glycémie, à trois états cachés : tendance à la baisse, tendance à la constance, tendance à la hausse.

On pourrait par ailleurs augmenter le nombre de modèles de niveau d'entrée ou de niveau(x) intermédiaire(s) en augmentant le nombre de capteurs.

Un modèle unique de niveau de sortie pourrait présenter cinq états cachés correspondant à cinq situations à détecter :

- « hyperglycémie à traiter » : glycémie très haute,
- « risque d'hyperglycémie à surveiller » : glycémie haute et à la hausse,
- « rien à signaler » : glycémie élevée mais constante ou normale ou basse et constante,
- « risque d'hypoglycémie à surveiller » : glycémie basse et à la baisse,
- « hypoglycémie à traiter » : glycémie très basse.

Il apparaîtra de façon plus générale à l'homme de l'art que diverses modifications peuvent être apportées aux modes de réalisation décrits ci-dessus, à la lumière de l'enseignement qui vient de lui être divulgué. Dans les revendications qui suivent, les termes utilisés ne doivent pas être interprétés comme limitant les revendications aux modes de réalisation exposés dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

**Revendications**

**1.** Dispositif (20) de détection d'au moins une situation (S ; S-j) parmi un ensemble de situations prédéterminées correspondant à des états dans lesquels est susceptible de se trouver un système physique (50) tel qu'un objet inanimé, une personne ou un animal observé par au moins un capteur (28), à partir de données d'observation du système physique fournies par le capteur, comportant :

- au moins un capteur (28) pour la fourniture d'une séquence (M) de données d'observation du système physique,
- des moyens (30) de stockage d'au moins un modèle statistique (HMM) associant des valeurs possibles des données d'observation aux situations prédéterminées,
- un calculateur (32), relié au capteur (28) et aux moyens de stockage (30), programmé pour sélectionner au moins l'une (S ; S-j) des situations, parmi la pluralité de situations prédéterminées, à partir de la séquence (M) de données d'observation et dudit au moins un modèle statistique (HMM),

dans lequel les moyens de stockage (30) comportent une pluralité de modèles statistiques répartis en plusieurs niveaux (L-1, L-i, L-N) ordonnés entre :

- un premier niveau (L-1), dit niveau d'entrée, dans lequel chaque modèle statistique associe des valeurs possibles d'au moins une partie des données d'observation à des états intermédiaires propres à ce modèle statistique, et
- un dernier niveau (L-N), dit niveau de sortie, dans lequel chaque modèle statistique associe des valeurs possibles d'au moins une partie des états intermédiaires d'un niveau inférieur (L-1, L-i) à au moins une partie des situations prédéterminées,

et tels que chaque modèle statistique de niveau autre que le niveau de sortie est conçu pour présenter des états intermédiaires dont il est possible d'extraire une séquence de valeurs à partir d'une séquence d'au moins une partie des données d'observation et/ou d'une séquence de valeurs d'états intermédiaires d'un niveau inférieur qu'il reçoit, le calculateur (32) étant programmé (36, 38) pour :

- extraire au moins une séquence de valeurs d'états intermédiaires d'au moins un modèle statistique de niveau autre que le niveau de sortie, et
- sélectionner ladite au moins une situation à partir au moins du ou des modèles statistiques du niveau de sortie (L-N) associant ladite au moins une situation aux valeurs possibles des états intermédiaires de ladite au moins une séquence extraite,

**caractérisé en ce que** le calculateur (32) est en outre programmé (36, 38) pour normaliser ladite au moins une séquence de valeurs d'états intermédiaires extraite en imposant que, pour chaque modèle statistique dont est extraite ladite au moins une séquence de valeurs d'états intermédiaires, la somme à chaque instant des valeurs d'états intermédiaires de la séquence correspondante soit égale à une constante indépendante du temps.

**2.** Dispositif de détection (20) selon la revendication 1, dans lequel le calculateur (32) est programmé (36, 38) pour déterminer une séquence de valeurs normalisées de tous les états intermédiaires à partir de tous les modèles statistiques définis pour déterminer ces états intermédiaires et de valeurs que ces modèles statistiques reçoivent.

**3.** Dispositif de détection (20) selon la revendication 1 ou 2, dans lequel les valeurs normalisées sont des valeurs de probabilités.

**4.** Dispositif de détection (20) selon la revendication 3, dans lequel au moins un modèle statistique (HMM) de niveau autre que le niveau de sortie est un modèle de Markov à états cachés et dans lequel le calculateur (32) est programmé (36, 38) pour déterminer de façon itérative une séquence de valeurs de probabilités d'au moins une partie de ses états cachés à partir d'une séquence de données fournies en entrée de ce modèle de Markov selon une relation de récurrence entre valeurs successives de la séquence de valeurs de probabilités, cette relation de récurrence faisant intervenir une unique valeur, dépendante de l'indice de récurrence, de la séquence de données fournies en entrée.

**5.** Dispositif de détection (20) selon la revendication 4, dans lequel la relation de récurrence prend la forme suivante :

INITIALISATION :

$$\tilde{p}\big(X_0 = i \big| Y_0\big) = p\big(X_0 = i\big) p\big(Y_0 \big| X_0 = i\big)$$

et

$$p\big(X_0 = i \big| Y_0\big) = \frac{\tilde{p}\big(X_0 = i \big| Y_0\big)}{\sum_j \tilde{p}\big(X_0 = j \big| Y_0\big)},$$

RECURRENCE SUR N :

$$\tilde{p}\big(X_{N+1} = i \big| Y_{0:N+1}\big) = \sum_j p\big(X_N = j \big| Y_{0:N}\big) p\big(Y_{N+1} \big| X_{N+1} = i\big) p\big(X_{N+1} = i \big| X_N = j\big)$$

et

$$p\big(X_{N+1} = i \big| Y_{0:N+1}\big) = \frac{\tilde{p}\big(X_{N+1} = i \big| Y_{0:N+1}\big)}{\sum_j \tilde{p}\big(X_{N+1} = j \big| Y_{0:N+1}\big)},$$

où i représente l'indice de l'état caché considéré, N l'indice de séquence, Y le processus statistique représentant la séquence de données fournies en entrée, X le processus statistique correspondant à la séquence d'états cachés la plus adaptée à la séquence de données d'entrée.

**6.** Dispositif de détection (20) selon l'une quelconque des revendications 1 à 5, dans lequel :

- le niveau de sortie (L-N) comporte un modèle statistique de Markov (HMM-N) dont les états cachés correspondent aux situations prédéterminées et dont les observations sont choisies parmi des valeurs d'états intermédiaires estimées et fournies par le calculateur (32), et
- le calculateur (32) est programmé pour fournir en sortie une séquence (S) de situations, chacune sélectionnée parmi les situations prédéterminées, à partir de la séquence de valeurs d'états intermédiaires et du modèle statistique de Markov (HMM-N) du niveau de sortie.

**7.** Dispositif de détection (20) selon l'une quelconque des revendications 1 à 5, dans lequel :

- le niveau de sortie (L-N) comporte un modèle statistique de Markov à états cachés (HMM-N,1 ..., HMM-N,p) pour chaque situation de l'ensemble de situations prédéterminées, dont les observations sont choisies parmi des valeurs d'états intermédiaires estimées et fournies par le calculateur (32), et

- le calculateur (32) est programmé pour sélectionner un modèle de Markov (HMM-N,1 ..., HMM-N,p) du niveau de sortie à partir de la séquence de valeurs d'états intermédiaires et pour fournir en sortie la situation correspondante (S-j).

8. Dispositif de détection (20) selon l'une quelconque des revendications 1 à 7, dans lequel au moins un modèle statistique (HMM-i,1 ..., HMM-i,n) de niveau (L-i) autre que le niveau d'entrée (L-1) et le niveau de sortie (L-N) est un modèle de Markov à états cachés et à observations choisis au moins en partie parmi les états intermédiaires.

9. Dispositif de détection (20) selon l'une quelconque des revendications 1 à 8, dans lequel au moins un modèle statistique est à observations choisies en partie parmi les valeurs de données d'observation et en partie parmi des valeurs d'états intermédiaires d'un autre modèle statistique estimées et fournies par le calculateur (32).

10. Procédé de détection d'au moins une situation (S ; S-j) parmi un ensemble de situations prédéterminées correspondant à des états dans lesquels est susceptible de se trouver un système physique (50) tel qu'un objet inanimé, une personne ou un animal observé par au moins un capteur (28), à partir de données d'observation du système physique fournies par le capteur, comportant les étapes suivantes :

- réception (100) d'une séquence (M) de données d'observation du système physique (50) fournie par le capteur (28),
- sélection (104, 106, 108) d'au moins l'une (S ; S-j) des situations, parmi la pluralité de situations prédéterminées, à partir de la séquence (M) de données d'observation et d'au moins un modèle statistique (HMM) stocké en mémoire (30) et associant des valeurs possibles des données d'observation aux situations prédéterminées,

dans lequel, la mémoire (30) stockant une pluralité de modèles statistiques (HMM) répartis en plusieurs niveaux ordonnés (L-1, L-i, L-N), la sélection comporte les étapes suivantes :

- détermination (104, 106) de valeurs d'états intermédiaires à partir de la séquence (M) de données d'observation et d'au moins un modèle statistique d'un premier niveau (L-i), dit niveau d'entrée, dans lequel chaque modèle statistique associe des valeurs possibles d'au moins une partie des données d'observation à des états intermédiaires propres à ce modèle statistique, et
- sélection (108) de ladite au moins une situation (S ; S-j), à partir des valeurs d'états intermédiaires et d'au moins un modèle statistique d'un dernier niveau (L-N), dit niveau de sortie, dans lequel chaque modèle statistique associe des valeurs possibles d'au moins une partie des états intermédiaires d'un niveau inférieur à au moins une partie des situations prédéterminées,

chaque modèle statistique de niveau autre que le niveau de sortie étant conçu pour présenter des états intermédiaires dont il est possible d'extraire une séquence de valeurs à partir d'une séquence d'au moins une partie des données d'observation et/ou d'une séquence de valeurs d'états intermédiaires d'un niveau inférieur qu'il reçoit, le procédé comportant en outre les étapes suivantes :

- extraire au moins une séquence de valeurs d'états intermédiaires d'au moins un modèle statistique de niveau autre que le niveau de sortie,
- normaliser ladite au moins une séquence de valeurs d'états intermédiaires extraite en imposant que, pour chaque modèle statistique dont est extraite ladite au moins une séquence de valeurs d'états intermédiaires, la somme à chaque instant des valeurs d'états intermédiaires de la séquence correspondante soit égale à une constante indépendante du temps, et
- sélectionner ladite au moins une situation à partir au moins du ou des modèles statistiques du niveau de sortie (L-N) associant ladite au moins une situation aux valeurs possibles des états intermédiaires de ladite au moins une séquence extraite et normalisée.

11. Programme d'ordinateur (36) téléchargeable depuis un réseau de communication et/ou enregistré sur un support lisible par ordinateur et/ou exécutable par un processeur (34), **caractérisé en ce qu'**il comprend des instructions pour l'exécution des étapes d'un procédé de détection selon la revendication 10, lorsque ledit programme est exécuté sur un ordinateur.

**Patentansprüche**

1. Vorrichtung (20) zum Detektieren mindestens einer Situation (S; S-j) aus einer Einheit von vorbestimmten Situationen, die Zuständen entsprechen, in denen sich ein physikalisches System (50), wie ein unbelebtes Objekt, eine Person oder ein durch mindestens einen Sensor (28) beobachtetes Tier befinden kann, ausgehend von Beobachtungsdaten des physikalischen Systems, die von dem Sensor bereitgestellt werden, Folgendes beinhaltend:

   - mindestens einen Sensor (28) für die Bereitstellung einer Sequenz (M) von Beobachtungsdaten des physikalischen Systems,
   - Mittel (30) zum Speichern mindestens eines statistischen Modells (HMM), welches mögliche Werte der Beobachtungsdaten vorbestimmten Situationen zuordnet,
   - einen Rechner (32), der mit dem Sensor (28) und den Mitteln (30) zum Speichern verbunden ist, der programmiert ist, um mindestens eine (S; S-j) der Situationen aus der Vielzahl von vorbestimmten Situationen ausgehend von der Sequenz (M) von Beobachtungsdaten und dem mindestens einen statistischen Modell (HMM) auszuwählen,

   wobei die Mittel (30) zum Speichern eine Vielzahl von statistischen Modellen beinhalten, die in mehrere Stufen (L-1, L-i, L-N) aufgeteilt sind, geordnet zwischen:

   - einer ersten Stufe (L-1), die Eingangsstufe genannt wird, bei der jedes statistische Modell mögliche Werte mindestens eines Teils der Beobachtungsdaten Zwischenzuständen zuordnet, die diesem statistischen Modell eigen sind, und
   - einer letzten Stufe (L-N), die Ausgangsstufe genannt wird, bei der jedes statistische Modell mögliche Werte mindestens eines Teils der Zwischenzustände einer unteren Stufe (L-1, L-i) mindestens einem Teil der vorbestimmten Situationen zuordnet,

   und so, dass jedes statistische Modell einer anderen Stufe als der Ausgangsstufe gestaltet ist, um Zwischenzustände aufzuweisen, aus denen es möglich ist, eine Sequenz von Werten ausgehend von einer Sequenz von mindestens einem Teil der Beobachtungsdaten und/oder einer Sequenz von Zwischenzustandswerten einer Stufe zu extrahieren, die niedriger als jene ist, die sie empfängt,
   wobei der Rechner (32) programmiert (36, 38) ist, um:

   - mindestens eine Sequenz von Zwischenzustandswerten mindestens eines statistischen Modells einer anderen Stufe als der Ausgangsstufe zu extrahieren, und
   - die mindestens eine Situation ausgehend von mindestens dem oder den statistischen Modellen der Ausgangsstufe (L-N) auszuwählen, welche die mindestens eine Situation den möglichen Werten der Zwischenzustände der mindestens einen extrahierten Sequenz zuordnet,

   **dadurch gekennzeichnet, dass** der Rechner (32) weiter programmiert (36, 38) ist, um die mindestens eine extrahierte Sequenz von Zwischenzustandswerten durch das Durchsetzen zu normalisieren, dass für jedes statistische Modell, aus dem die mindestens eine Sequenz von Zwischenzustandswerten extrahiert ist, die Summe zu jedem Zeitpunkt der Zwischenzustandswerte der entsprechenden Sequenz gleich einer von der Zeit unabhängigen Konstanten ist.

2. Vorrichtung (20) zum Detektieren nach Anspruch 1, wobei der Rechner (32) programmiert (36, 38) ist, um eine Sequenz von normalisierten Werten aus allen Zwischenzuständen ausgehend von allen statistischen Modellen zu bestimmen, die definiert sind, um diese Zwischenzustände zu bestimmen, und aus Werten, die diese statistischen Modelle empfangen.

3. Vorrichtung (20) zum Detektieren nach Anspruch 1 oder 2, wobei die normalisierten Werte Wahrscheinlichkeitswerte sind.

4. Vorrichtung (20) zum Detektieren nach Anspruch 3, wobei mindestens ein statistisches Modell (HMM) einer anderen Stufe als der Ausgangsstufe ein Markov-Modell mit verborgenen Zuständen ist, und wobei der Rechner (32) programmiert (36, 38) ist, um iterativ eine Sequenz von Wahrscheinlichkeitswerten von mindestens einem Teil seiner verborgenen Zustände ausgehend von einer Sequenz von Daten zu bestimmen, die am Eingang dieses Markov-Modells entsprechend einer Wiederholungsbeziehung zwischen den aufeinanderfolgenden Werten der Sequenz von Wahrscheinlichkeitswerten bereitgestellt werden, wobei diese Wiederholungsbeziehung einen einzigen Wert, der von dem Wiederholungsindex abhängig ist, der Sequenz von am Eingang bereitgestellten Daten einsetzt.

5. Vorrichtung (20) zum Detektieren nach Anspruch 4, wobei die Wiederholungsbeziehung die folgende Form annimmt:

Initialisierung:

$$\tilde{p}(X_0 = i|Y_0) = p(X_0 = i)p(Y_0|X_0 = i)$$

und

$$p(X_0 = i|Y_0) = \frac{\tilde{p}(X_0 = i|Y_0)}{\sum_j \tilde{p}(X_0 = j|Y_0)},$$

Wiederholung auf N:

$$\tilde{p}(X_{N+1} = i|Y_{0:N+1}) = \sum_i p(X_N = j|Y_{0:N})p(Y_{N+1}|X_{N+1} = i)p(X_{N+1} = i|X_N = j)$$

und

$$p(X_{N+1} = i|Y_{0:N+1}) = \frac{\tilde{p}(X_{N+1} = i|Y_{0:N+1})}{\sum_j \tilde{p}(X_{N+1} = j|Y_{0:N+1})},$$

wobei i den Index des angenommenen verborgenen Zustands darstellt, N den Sequenzindex, Y den statistischen Prozess, der die Sequenz von am Eingang bereitgestellten Daten darstellt, X den statistischen Prozess, welcher der Sequenz von verborgenen Zuständen entspricht, die sich am besten für die Sequenz von Eingangsdaten eignet.

6. Vorrichtung (20) zum Detektieren nach einem der Ansprüche 1 bis 5, wobei:

- die Ausgangsstufe (L-N) ein statistisches Markov-Modell (HMM-N) beinhaltet, dessen verborgene Zustände den vorbestimmten Situationen entsprechen, und deren Beobachtungen aus den geschätzten Zwischenzustandswerten ausgewählt werden, und durch den Rechner (32) bereitgestellt werden, und
- der Rechner (32) programmiert ist, um am Ausgang eine Sequenz (S) von Situationen bereitzustellen, von denen jede aus den vorbestimmten Situationen ausgehend von der Sequenz von Zwischenzustandswerten und dem statistischen Markov-Modell (HMM-N) der Ausgangsstufe ausgewählt wird.

7. Vorrichtung (20) zum Detektieren nach einem der Ansprüche 1 bis 5, wobei:

- die Ausgangsstufe (L-N) ein statistisches Markov-Modell mit verborgenen Zuständen (HMM-N, 1 ..., HMM-N, p) für jede Situation der Einheit von vorbestimmten Situationen beinhaltet, deren Beobachtungen aus den geschätzten Zwischenzustandswerten ausgewählt werden und durch den Rechner (32) bereitgestellt werden, und
- der Rechner (32) programmiert ist, um ein Markov-Modell (HMM-N, 1 ..., HMM-N, p) der Ausgangsstufe ausgehend von der Sequenz von Zwischenzustandswerten auszuwählen, und um am Ausgang die entsprechende Situation (S-j) bereitzustellen.

8. Vorrichtung (20) zum Detektieren nach einem der Ansprüche 1 bis 7, wobei mindestens ein statistisches Modell (HMM-i, 1 ..., HMM-i, n) einer anderen Stufe (L-i) als der Eingangsstufe (L-1) und der Ausgangsstufe (L-N) ein Markov-Modell mit verborgenen Zuständen und mit Beobachtungen ist, die mindestens teilweise aus den Zwischenzuständen ausgewählt werden.

9. Vorrichtung (20) zum Detektieren nach einem der Ansprüche 1 bis 8, wobei mindestens ein statistisches Modell eines mit Beobachtungen ist, die teilweise aus den Beobachtungsdatenwerten und teilweise aus den Zwischenzustandswerten eines anderen statistischen Modells ausgewählt werden, die geschätzt werden und von dem Rechner (32) bereitgestellt werden.

10. Verfahren zum Detektieren mindestens einer Situation (S; S-j) aus einer Einheit von vorbestimmten Situationen, die Zuständen entsprechen, in denen sich ein physikalisches System (50), wie ein unbelebtes Objekt, eine Person oder ein durch mindestens einen Sensor (28) beobachtetes Tier befinden kann, ausgehend von Beobachtungsdaten des physikalischen Systems, die von dem Sensor bereitgestellt werden, die folgenden Schritte beinhaltend:

- Empfangen (100) einer Sequenz (M) von Beobachtungsdaten des physikalischen Systems (50), die von dem Sensor (28) bereitgestellt wird,
- Auswählen (104, 106, 108) mindestens einer (S; S-j) der Situationen aus der Vielzahl von vorbestimmten Situationen ausgehend von der Sequenz (M) von Beobachtungsdaten und mindestens einem statistischen Modell (HMM), das im Speicher (30) gespeichert ist, und mögliche Werte der Beobachtungsdaten vorbestimmten Situationen zuordnet,

wobei der Speicher (30) eine Vielzahl von statistischen Modellen (HMM) speichert, die in mehrere geordnete Stufen (L-1, L-i, L-N) aufgeteilt sind, wobei das Auswählen die folgenden Schritte aufweist:

- Bestimmen (104, 106) von Zwischenzustandswerten ausgehend von der Sequenz (M) von Beobachtungsdaten und mindestens einem statistischen Modell einer ersten Stufe (L-i), die Eingangsstufe genannt wird, bei der jedes statistische Modell mögliche Werte mindestens eines Teils der Beobachtungsdaten Zwischenzuständen zuordnet, die diesem statistischen Modell eigen sind, und
- Auswählen (108) der mindestens einen Situation (S; S-j) ausgehend von den Zwischenzustandswerten und mindestens einem statistischen Modell einer letzten Stufe (L-N), die Ausgangsstufe genannt wird, bei der jedes statistische Modell mögliche Werte mindestens eines Teils der Zwischenzustände einer unteren Stufe mindestens einem Teil der vorbestimmten Situationen zuordnet,

wobei jedes statistische Modell einer anderen Stufe als der Ausgangsstufe gestaltet ist, um Zwischenzustände aufzuweisen, aus denen es möglich ist, eine Sequenz von Werten ausgehend von einer Sequenz von mindestens einem Teil der Beobachtungsdaten und/oder einer Sequenz von Zwischenzustandswerten einer Stufe zu extrahieren, die niedriger als jene ist, die sie empfängt,
wobei das Verfahren weiter die folgenden Schritte beinhaltet:

- Extrahieren mindestens einer Sequenz von Zwischenzustandswerten mindestens eines statistischen Modells einer anderen Stufe als der Ausgangsstufe,
- Normalisieren der mindestens einen extrahierten Sequenz von Zwischenzustandswerten, indem durchgesetzt wird, dass für jedes statistische Modell, aus dem die mindestens eine Sequenz von Zwischenzustandswerten extrahiert ist, die Summe zu jedem Zeitpunkt der Zwischenzustandswerte der entsprechenden Sequenz gleich einer von der Zeit unabhängigen Konstanten ist, und
- Auswählen der mindestens einen Situation ausgehend von mindestens dem oder den statistischen Modellen der Ausgangsstufe (L-N), welche die mindestens eine Situation den möglichen Werten der Zwischenzustände der mindestens einen extrahierten und normalisierten Sequenz zuordnet.

11. Computerprogramm (36), das aus einem Kommunikationsnetzwerk heruntergeladen und/oder auf einem Support gespeichert werden kann, der von einem Computer gelesen und/oder durch einen Prozessor (34) ausgeführt werden kann, **dadurch gekennzeichnet, dass** es Befehle zum Ausführen der Schritte eines Verfahrens zum Detektieren nach Anspruch 10 umfasst, wenn das Programm auf einem Computer ausgeführt wird.

**Claims**

1. Device (20) for detecting at least one scenario (S; S-j) from a set of predetermined scenarios corresponding to states wherein a physical system (50) such as an inanimate object, a person or an animal observed by at least one sensor (28) is likely to be found, on the basis of physical system observation data provided by the sensor, comprising:

- at least one sensor (28) for providing a physical system observation data sequence (M),
- means (30) for storing at least one statistical model (HMM) associating possible observation data values with the predetermined scenarios,
- a computer (32), connected to the sensor (28) and the storage means (30), programmed to select at least one (S; S-j) of the scenarios, from the plurality of predetermined scenarios, based on the observation data sequence (M) and said at least one statistical model (HMM),

wherein the storage means (30) comprise a plurality of statistical models broken down into a plurality of ordered levels (L-1, L-i, L-N) between:

- a first level (L-1), or input level, wherein each statistical model associates possible values of at least a portion of the observation data with specific intermediate states for the statistical model, and
- a final level (L-N), or output level, wherein each statistical model associates possible values of at least a portion of the intermediate states of a lower level (L-1, L-i) with at least a portion of the predetermined scenarios,

and such that each statistical model of a level other than the output level is designed to have intermediate states from which it is possible to extract a sequence of values based on a sequence of at least a portion of the observation data and/or a sequence of intermediate state values from a lower level that it receives,
the computer (32) being programmed (36, 38) for:

- extracting at least one sequence of intermediate state values from at least one statistical model of a level other than the output level, and
- selecting said at least one scenario at least based on the output level (L-N) statistical model(s) that associate(s) said at least one scenario with the possible intermediate state values of said at least one extracted sequence,

**characterized in that** the computer (32) is further programmed (36, 38) to standardize said at least one extracted sequence of intermediate state values by requiring that, for each statistical model from which said at least one sequence of intermediate state values is extracted, the sum at each instant of the intermediate state values of the corresponding sequence is equal to a time-independent constant.

2. Detection device (20) according to claim 1, wherein the computer (32) is programmed (36, 38) to determine a sequence of standardised values of all the intermediate states based on all the statistical models defined to determine the intermediate states and on values received by the statistical models.

3. Detection device (20) according to claim 1 or 2, wherein the standardised values are probability values.

4. Detection device (20) according to claim 3, wherein at least one statistical model (HMM) of a level other than the output level is a hidden Markov model and wherein the computer (32) is programmed (36, 38) to determine a sequence of probability values of at least a portion of the hidden states thereof iteratively on the basis of a data sequence provided at the input of the Markov model according to a recurrence relation between successive values of the probability value sequence, this recurrence relation involving a single value, dependent on the recurrence index, of the input data sequence provided.

5. Detection device (20) according to claim 4, wherein the recurrence relation takes the following form:

INITIALISATION:

$$\tilde{p}\left(X_0 = i \middle| Y_0\right) = p\left(X_0 = i\right) p\left(Y_0 \middle| X_0 = i\right)$$

and

$$p(X_0 = i|Y_0) = \frac{\tilde{p}(X_0 = i|Y_0)}{\sum_j \tilde{p}(X_0 = j|Y_0)},$$

RECURRENCE ON N:

$$\tilde{p}(X_{N+1} = i|Y_{0:N+1}) = \sum_j p(X_N = j|Y_{0:N})p(Y_{N+1}|X_{N+1} = i)p(X_{N+1} = i|X_N = j)$$

and

$$p(X_{N+1} = i|Y_{0:N+1}) = \frac{\tilde{p}(X_{N+1} = i|Y_{0:N+1})}{\sum_j \tilde{p}(X_{N+1} = j|Y_{0:N+1})},$$

where i represents the hidden state index in question, N the sequence index, Y the statistical process representing the input data sequence provided, X the statistical process corresponding to the most suitable hidden state sequence for the input data sequence.

6. Detection device (20) according to any of claims 1 to 5, wherein:

- the output level (L-N) comprises a Markov statistical model (HMM-N) wherein the hidden states correspond to the predetermined scenarios and wherein the observations are chosen from estimated intermediate state values provided by the computer (32), and
- the computer (32) is programmed to output a sequence (S) of scenarios, wherein each is selected from the predetermined scenarios, on the basis of the sequence of intermediate state values and the output level Markov statistical model (HMM-N).

7. Detection device (20) according to any of claims 1 to 5, wherein:

- the output level (L-N) comprises a hidden Markov statistical model (HMM-N,1 ..., HMM-N,p) for each scenario from the set of predetermined scenarios, wherein the observations are chosen from estimated intermediate state values provided by the computer (32), and
- the computer (32) is programmed to select an output level Markov model (HMM-N,1 ..., HMM-N,p) on the basis of the sequence of intermediate state values and to output the corresponding scenario (S-j).

8. Detection device (20) according to any of claims 1 to 7, wherein at least one statistical model (HMM-i,1 ..., HMM-i,n) of a different level (L-i) to the input level (L-1) and the output level (L-N) is a hidden Markov model having observations chosen at least in part from the intermediate states.

9. Detection device (20) according to any of claims 1 to 8, wherein at least one statistical model has observations chosen in part from the observation data values and in part from the intermediate state values of another statistical model estimated and provided by the computer (32).

10. Method for detecting at least one scenario (S; S-j) from a set of predetermined scenarios corresponding to states wherein a physical system (50) such as an inanimate object, a person or an animal observed by at least one sensor (28) is likely to be found, on the basis of physical system observation data provided by the sensor, comprising the following steps:

- receiving (100) a physical system (50) observation data sequence (M) provided by the sensor (28),
- selecting (104, 106, 108) at least one (S; S-j) of the scenarios, from the plurality of predetermined scenarios, on the basis of the observation data sequence (M) and at least one statistical model (HMM) stored in memory (30) and associating possible observation data values with the predetermined scenarios,

wherein, the memory (30) storing a plurality of statistical models (HMM) broken down into a plurality of ordered levels (L-1, L-i, L-N), the selection comprises the following steps:

- determining (104, 106) intermediate state values on the basis of the observation data sequence (M) and at least one first-level (L-i), or input level, statistical model, wherein each statistical model associates possible values of at least a portion of the observation data with specific intermediate states for the statistical model, and
- selecting (108) said at least one scenario (S; S-j), on the basis of the intermediate state values and at least one final-level (L-N), or output level, statistical model, wherein each statistical model associates possible values of at least a portion of the intermediate states from a lower level with at least a portion of the predetermined scenarios,

wherein each statistical model of a level other than the output level is designed to have intermediate states from which it is possible to extract a sequence of values based on a sequence of at least a portion of the observation data and/or a sequence of intermediate state values from a lower level that it receives
wherein the method further comprises the following steps:

- extracting at least one sequence of intermediate state values from at least one statistical model of a level other than the output level,
- standardizing said at least one extracted sequence of intermediate state values by requiring that, for each statistical model from which said at least one sequence of intermediate state values is extracted, the sum at each instant of the intermediate state values of the corresponding sequence is equal to a time-independent constant, and
- selecting said at least one scenario at least based on the output level (L-N) statistical model(s) that associate(s) said at least one scenario with the possible intermediate state values of said at least one extracted and standardized sequence.

**11.** Computer program (36) suitable for being downloaded from a communication network and/or saved on a computer-readable medium and/or executable by a processor (34), **characterised in that** it comprises instructions for executing steps of a method for detecting according to claim 10, when said program is executed on a computer.

## _Figure 1_

20

36

-38-    -40-

-28-

M

-34-    -32-

-42-

S    M

L-1

CNX1

HMM-1,1 · · · HMM-1,m

30

L-i

CNXi

HMM-i,1 · · · HMM-i,n

L-N

CNXN

HMM-N

-22-    -24-    -26-

50

20    52

## _Figure 3_

54

## *Figure 2*

20

## *Figure 4*

*Figure 5*

*Figure 6*

*Figure 7*

*Figure 8*

## Figure 9

## Figure 10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **P. JALLON et al.** Detection system of motor epileptic seizures through motion analysis with 3d accelerometers. *conférence IEEE EMBC,* 2009

- **L. RABINER.** A tutorial on Hidden Markov Models and selected applications in speech récognition. *Proceedings of the IEEE,* Février 1989, vol. 77 (2), 257-286